# EUROPÄISCHE PATENTSCHRIFT

(11) **EP 1 146 836 B1**
(45) Veröffentlichungstag und Bekanntmachung des Hinweises auf die Patenterteilung: **12.10.2005**
(21) Anmeldenummer: 00909110.9
(22) Anmeldetag: 27.01.2000
(51) Int. Cl.: A61F 2/44, A61F 2/46

(54) **ZWISCHENWIRBELIMPLANTAT**
INTERVERTEBRAL IMPLANT
IMPLANT INTERVERTEBRAL

(30) Priorität: 30.01.1999 DE 19903764
(43) Veröffentlichungstag der Anmeldung: 24.10.2001
(73) Patentinhaber: Aesculap AG & Co. KG, 78532 Tuttlingen (DE)
(72) Erfinder: FUSS, Franz, Konstantin, A-2700 Wiener Neustadt (AT); SABITZER, Ronald, J., A-1160 Wien (AT); ECKHOF, Stephan, D-78532 Tuttlingen (DE)
(74) Vertreter: Boehme, Ulrich
(86) Internationale Anmeldenummer: PCT/EP2000/000624
(87) Internationale Veröffentlichungsnummer: WO 2000/044317

(56) Entgegenhaltungen:
- EP-A- 0 697 200
- WO-A-97/23174
- WO-A-97/37619
- DE-C- 4 318 700
- DE-U- 29 720 022
- US-A- 5 397 364

## Beschreibung

Die Erfindung betrifft ein Zwischenwirbelimplantat mit einer oberen und einer unteren Stützfläche zur Anlage an benachbarten Wirbelkörpern, mit einer konkaven dorsalen Längsseite, einer dieser gegenüberliegenden ventralen Längsseite, mit einer abgerundeten Kontur an den beiden die Längsseiten verbindenden Stirnseiten und mit mindestens einer das Implantat von der oberen zur unteren Stützfläche durchsetzenden Durchbrechung.

Ein solches Zwischenwirbelimplantat ist beispielsweise aus der DE 297 20 022 U1 bekannt. Es dient dazu, nach Entfernung einer Bandscheibe den Zwischenwirbelraum zwischen zwei benachbarten Wirbelkörpern teilweise auszufüllen und dadurch die benachbarten Wirbelkörper gegeneinander abzustützen. Neben der Stützfunktion sollen diese Implantate auch eine knöcherne Verbindung benachbarter Wirbelkörper ermöglichen, daher füllen sie den Zwischenwirbelraum nur teilweise aus, bei der aus der DE 297 20 022 U1 bekannten Konstruktion wird das Implantat aus relativ schmalen Seitenwänden gebildet, die mit ihrer oberen und ihrer unteren Kante nur eine geringe Auflagefläche für die benachbarten Wirbelkörper ausbilden. Wenn ein Zwischenwirbelimplantat verwendet wird, das im wesentlichen als solider Körper ausgebildet ist und nur Durchbrechungen zur Aufnahme des Knochenmaterials aufweist, ist es häufig schwierig, das Knochenmaterial in diese Durchbrechungen und in verbleibende Freiräume des Zwischenwirbelraumes einzufüllen.

Es ist Aufgabe der Erfindung, ein gattungsgemäßes Zwischenwirbelimplantat so auszubilden, daß auch nach dem Einsetzen eines Zwischenwirbelimplantates in den Zwischenwirbelraum noch in einfacher Weise das Einfüllen von Knochenmaterial in nicht von dem Implantat eingenommene Bereiche des Zwischenwirbelraumes möglich ist.

Diese Aufgabe wird bei einem Zwischenwirbelimplantat der eingangs beschriebenen Art erfindungsgemäß dadurch gelöst, daß mindestens ein das Implantat durchsetzender Einführkanal für Knochenmaterial in dem Teil einer Stirnseite in das Implantat eintritt, der an die dorsale Längsseite anschließt, und im Bereich des Mittelabschnittes der ventralen Längsseite wieder aus dem Implantat austritt, wobei der Einführkanal in einer Ebene verläuft, die zwischen der oberen Stützfläche und der unteren Stützfläche angeordnet ist, und gegenüber einer Längsachse des Implantates in einer im wesentlichen parallel zu den Stützflächen verlaufenden Ebene zwischen 30° und 80° geneigt ist.

Günstig ist es, wenn der Einführkanal in dem Teil einer Stirnseite in das Implantat eintritt, der an die dorsale Längsseite anschließt. Dadurch kann Knochenmaterial in lateral-dorsaler Richtung in das Implantat eingeführt werden und kann von der Einführseite durch das Implantat hindurch auf die ventrale Seite des Zwischenwirbelraumes gelangen, der Zugang wird auf diese Weise also erheblich vereinfacht.

Besonders vorteilhaft ist es, wenn der Einführkanal eine das Implantat durchsetzende Durchbrechung durchquert. Auf diese Weise kann der Einführkanal auch verwendet werden, um Knochenmaterial in die Durchbrechung einzufüllen.

Bei einer besonders bevorzugten Ausführungsform der Erfindung ist vorgesehen, daß der Einführkanal an seinem ventralen Ende Befestigungselemente für ein in den Einführkanal eingeschobenes Instrument aufweist. Damit wird dem Einführkanal eine Doppelfunktion zugewiesen, einerseits dient er nämlich zum Befüllen des Zwischenwirbelraumes mit Knochenmaterial, zum anderen kann er ein Instrument aufnehmen, das beispielsweise dazu dient, das Zwischenwirbelimplantat zu handhaben und in den Zwischenwirbelraum einzuschieben. Durch die Befestigungselemente kann das Instrument fest mit dem Implantat verbunden werden, so daß der Chirurg beim Einsetzen das Implantat feinfühlig führen kann.

Als Befestigungselement könnte beispielsweise eine Bajonettverriegelung dienen, besonders vorteilhaft ist es, wenn das Befestigungselement durch ein Innengewinde gebildet wird.

Dabei kann der Einführkanal gegenüber einer Längsachse des Implantates in einer im wesentlichen parallel zu den Stützflächen verlaufenden Ebene zwischen 30° und 80° geneigt sein.

Bei einer bevorzugten Ausführungsform treten zwei Einführkanäle von verschiedenen Stirnseiten des Implantates in dieses ein, so daß das Einführen von Knochenmaterial und/oder das Handhaben des Implantates durch Einsetzen eines Handhabungsinstrumentes von beiden Endseiten her erfolgen kann.

Es ist besonders günstig, wenn das Implantat symmetrisch zu einer senkrecht auf den Stützflächen und auf den Längsseiten stehenden Mittelebene ausgebildet ist, dadurch läßt sich das Implantat in zwei Orientierungen einführen.

Bei einer bevorzugten Ausführungsform ist vorgesehen, daß zwei Durchbrechungen an den beiden Enden des Implantates angrenzend an dessen Stirnseite angeordnet sind.

Insbesondere können die Durchbrechungen einen kreisförmigen Querschnitt aufweisen.

Es ist günstig, wenn die Stützflächen gegeneinander geneigt sind, beispielsweise um einen Winkel zwischen 0° und 15°.

Weiterhin kann vorgesehen sein, daß die Stützflächen Vorsprünge tragen, die zur Fixierung an den anliegenden Wirbelkörpern beitragen.

Diese Vorsprünge können beispielsweise dornförmige Spitzen oder Rippen sein.

Besonders günstig ist es, wenn die Vorsprünge in Form von Rippen längs der Kanten der Durchbrechungen verlaufen. Die Durchbrechungen sind also mit einem rippenförmigen Kragen umgeben, der noch Einkerbungen zur besseren Verbindung mit den benachbarten Wirbelkörpern aufweisen kann.

Bei einer anderen Ausführungsform ist vorgesehen, daß die Vorsprünge Mikrovorsprünge sind, die durch eine rauhe Oberflächenstruktur der Stützflächen ausgebildet werden. Zu diesem Zweck können die Stützflächen zum Beispiel mit einer Beschichtung versehen sein, durch die eine aufgerauhte, Mikroporen aufweisende Oberflächenstruktur entsteht.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht eines Zwischenwirbelimplantates;
- Figur 2:: eine Draufsicht auf das Implantat der Figur 1;
- Figur 3:: eine Seitenansicht des Implantates der Figur 1 in Richtung des Pfeiles A in Figur 2;
- Figur 4:: eine Seitenansicht des Implantates der Figur 1 in Richtung des Pfeiles B in Figur 2;
- Figur 5:: eine Schnittansicht längs Linie 5-5 in Figur 4;
- Figur 6:: eine Darstellung des Einsetzvorganges des Implantates der Figuren 1 bis 3 mit Hilfe eines Einsetzwerkzeuges und
- Figur 7:: eine Darstellung der Einfüllung von Knochenmaterial mittels eines Einfüllinstrumentes durch das eingesetzte Implantat hindurch.

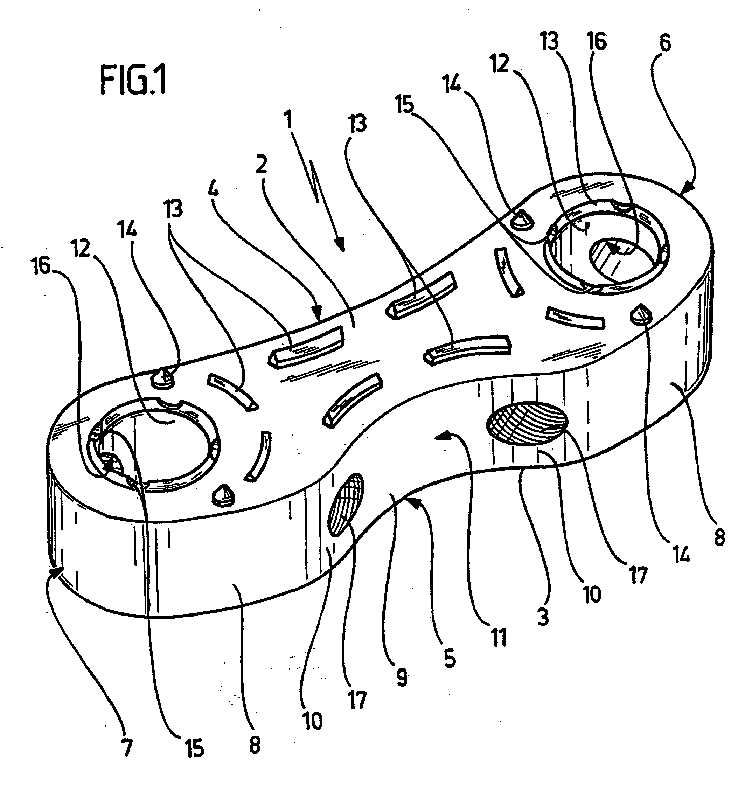

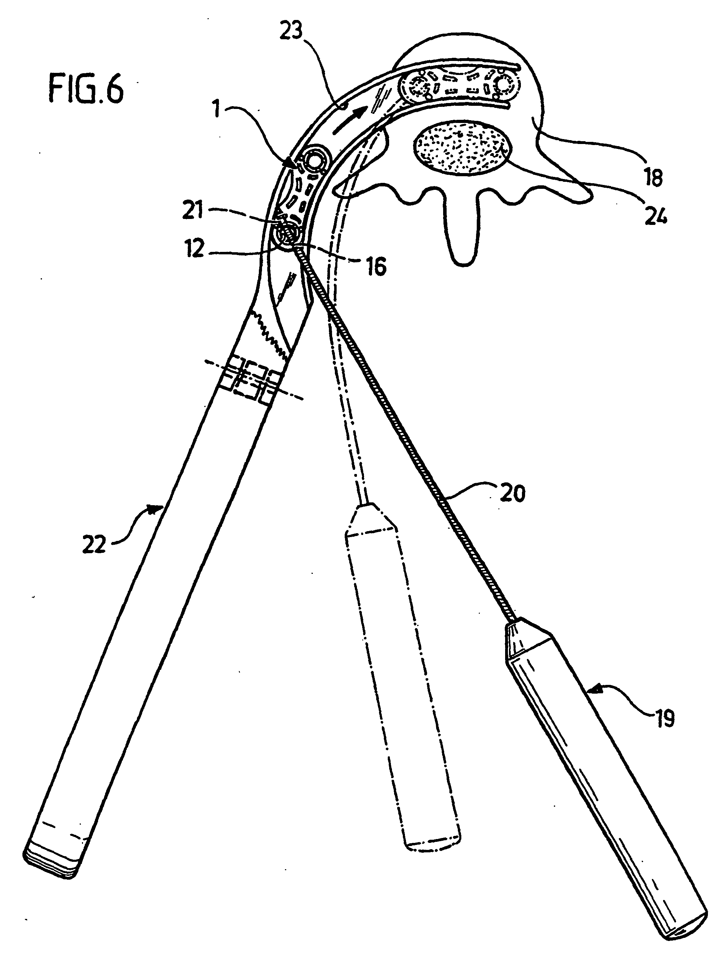

Das in den Figuren 1 bis 5 dargestellte Zwischenwirbelimplantat 1 ist plattenförmig ausgebildet und weist eine ebene obere Stützfläche 2 und eine ebene untere Stützfläche 3 auf. Seitlich wird das Zwischenwirbelimplantat 1 begrenzt durch eine senkrechte Seitenfläche, die sich unterteilt in eine dorsale Längsseite 4, in eine ihr gegenüberliegende ventrale Längsseite 5 und in zwei diese verbindende Stirnseiten 6, 7.

Die dorsale Längsseite 4 ist geringfügig konkav ausgebildet und erstreckt sich in einem im wesentlichen gleichmäßigen Bogen über den größten Teil der Länge des Zwischenwirbelimplantates 1, der Radius dieses Bogens liegt zwischen 30 mm und 50 mm.

An die beiden Endseiten der dorsalen Längsseite 4 schließen sich die kreisbogenförmig konturierten Stirnseiten 6 und 7 an, die sich im wesentlichen über jeweils einen Halbkreis erstrecken und die stetig und ohne Knick in die ventrale Längsseite 5 übergehen.

Die ventrale Längsseite 5 unterteilt sich in zwei außen liegende Seitenabschnitte 8, die sich an die Stirnseiten 6, 7 stetig und ohne Knick anschließen und die im wesentlichen parallel zu den gegenüberliegenden Teilen der dorsalen Längsseite 4 verlaufen, und in einen zwischen den beiden Seitenabschnitten 8 angeordneten Mittelabschnitt 9, der eine konkave, bogenförmige Kontur aufweist und sich über konvexe bogenförmige Zwischenabschnitte 10 in die Seitenabschnitte 8 fortsetzt. Auch hier sind alle Übergänge stetig und knickfrei, der Radius des konkaven Mittelabschnittes 9 liegt beispielsweise zwischen 5 mm und 15 mm.

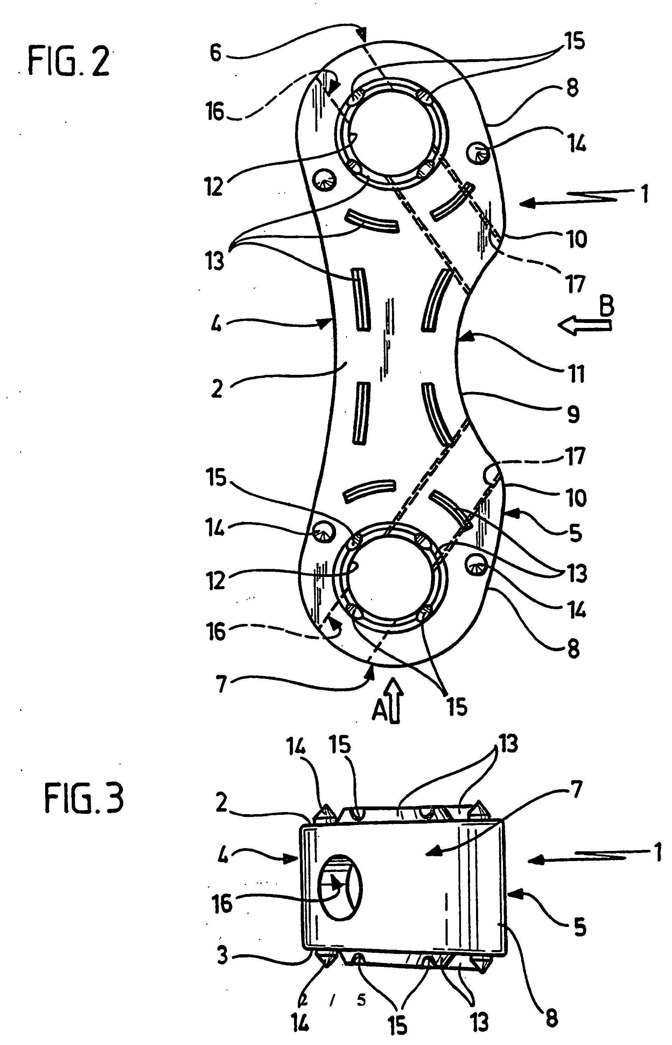

Auf diese Weise erhält das Zwischenwirbelimplantat 1 eine langgestreckte, insgesamt gebogene Form mit einem eine zurückspringende Ausnehmung 11 bildenden Mittelabschnitt 9, d.h. das Zwischenwirbelimplantat 1 ist an seinen Enden breiter als in seiner Mitte (Figur 2).

Konzentrisch zu den kreisbogenförmigen Stirnseiten 6 und 7 durchsetzen das Zwischenwirbelimplantat 1 im Querschnitt kreisförmige Durchbrechungen 12, deren Durchmesser liegt zwischen 4 mm und 8 mm.

Die obere Stützfläche 2 und die untere Stützfläche 3 sind geringfügig gegeneinander geneigt, beispielsweise um einen Winkel zwischen 0° und 15° (Figur 3), so daß der Abstand der oberen Stützfläche 2 von der unteren Stützfläche 3 an der dorsalen Längsseite 4 kleiner ist als an der ventralen Längsseite 5.

Die in sich ebenen Stützflächen 2 und 3 tragen Vorsprünge in Form von Rippen 13 und von dornförmigen Spitzen 14, teilweise verlaufen diese Rippen 13 längs der Kanten der Durchbrechungen 12, so daß die Durchbrechungen 12 von den Rippen 13 kragenförmig umgeben sind. In den Rippen 13 können sich Vertiefungen 15 befinden, die die Rippen 13 in einzelne Abschnitte unterteilen.

Beide Durchbrechungen 12 werden diametral durchsetzt von je einem Einführkanal 16, der jeweils an einer Stirnseite 6 bzw. 7 in das Zwischenwirbelimplantat 1 eintritt und im Bereich des konkav ausgebildeten Mittelabschnittes 9 wieder aus diesem austritt. Die Einführkanäle verlaufen in einer Ebene, die zwischen der oberen Stützfläche 2 und der unteren Stützfläche 3 angeordnet ist und im wesentlichen parallel zu diesen Stützflächen positioniert ist, gegenüber einer Längsachse des Zwischenwirbelimplantates 1, die beispielsweise durch die Mittelpunkte der beiden Durchbrechungen 12 definiert wird, schließen die Einführkanäle 16 einen Winkel zwischen 20° und 120° ein.

Im Eintrittsbereich, also an ihrem stirnseitigen Ende, weisen die Einführkanäle 16 eine glatte Innenwand auf, an der Austrittsseite, also an ihrem ausnehmungsseitigen Ende, sind sie mit einem Innengewinde 17 versehen.

Das Zwischenwirbelimplantat 1 besteht vorzugsweise aus einem körperverträglichen Metall, beispielsweise aus Titan oder einer Titanlegierung, oder aus Kunststoff.

Zum Einsetzen des beschriebenen Zwischenwirbelimplantates 1 in den Zwischenwirbelraum, also in den Spalt zwischen benachbarten Wirbelkörpern 18, wird das Zwischenwirbelimplantat 1 mit einem Einsetzinstrument 19 verbunden, welches beispielsweise einen flexiblen Schaft 20 mit einem Außengewinde 21 an seinem Ende aufweist. Dieser Schaft 20 wird in einen Einführkanal 16 eingesetzt und mittels seines Außengewindes 21 mit dessen Innengewinde 17 verschraubt, so daß das Zwischenwirbelimplantat 1 und das Einsetzinstrument 19 eine Einheit bilden.

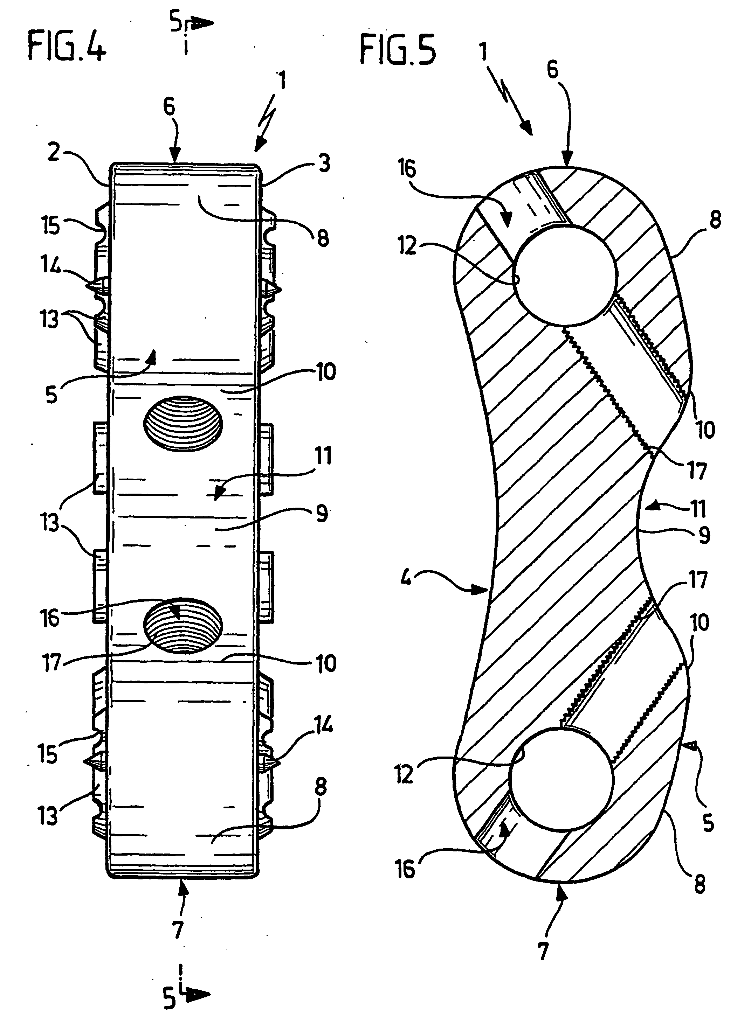

Längs eines Führungsinstrumentes 22, welches dorsallateral in den Körper eingeführt und mit seinem freien Ende im Zwischenwirbelraum positioniert wird, wird das Zwischenwirbelimplantat 1 längs der Führungsbahn 23 des Führungsinstrumentes 22 mit Hilfe des Einsetzinstrumentes 19 so weit vorgeschoben, bis es mit der dorsalen Längsseite 4 dicht am Wirbelkanal 24 des Wirbelkörpers 18 anliegend positioniert ist. Sobald diese Lage erreicht ist, kann das Führungsinstrument 22 aus dem Körper herausgezogen werden, der Schaft 20 des Führungsinstrumentes 22 wird aus dem Innengewinde 17 herausgedreht, und dann kann auch das Einsetzinstrument 19 wieder aus dem Körper herausgezogen werden (Figur 4).

Im eingesetzten Zustand befindet sich das Zwischenwirbelimplantat 1 dicht am Wirbelkanal 24 und bedeckt nur einen Teil der Fläche des Zwischenwirbelraumes zwischen benachbarten Wirbelkörpern 18.

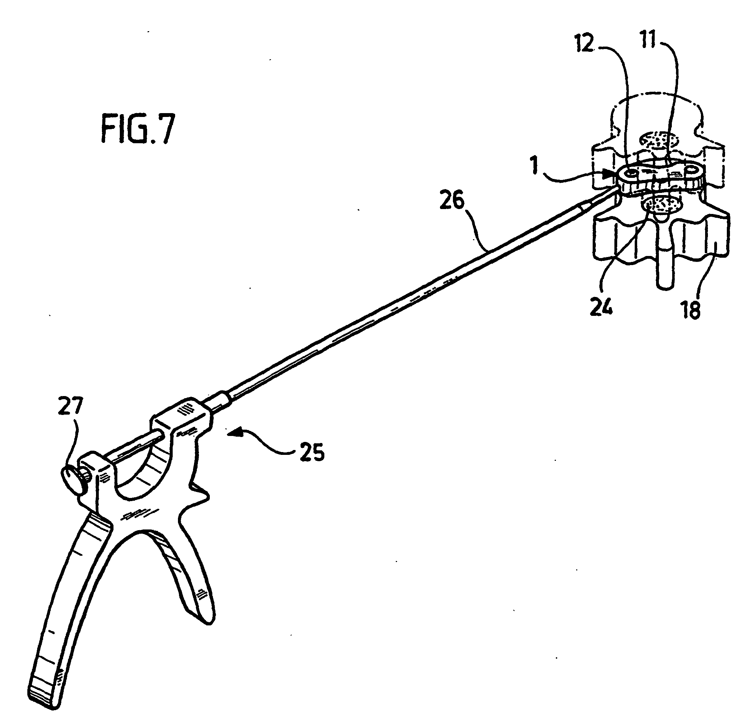

Nach dem Einsetzen des Zwischenwirbelimplantates 1 in der beschriebenen Weise wird ein Füllinstrument 25 mit einem länglichen und dünnen Füllrohr 26 ebenfalls lateral-dorsal in den Körper eingeführt und in den Einführkanal 16 eingeschoben (Figur 7). Durch das Einfüllrohr 26 wird mittels einer geeigneten Vorrichtung, beispielsweise mittels eines Stößels 27, Knochenmaterial aus dem Einfüllrohr 26 durch den Einführkanal 16 in den Körper eingefüllt und füllt dabei zunächst die zurückspringende Ausnehmung 11 auf und den angrenzenden Bereich des Zwischenwirbelraumes, außerdem kann mit diesem Instrument auch die jeweilige Durchbrechung 12 aufgefüllt werden, durch die der Einführkanal 16 hindurchtritt.

Das eingefüllte Knochenmaterial verfestigt sich und bildet sowohl in der Durchbrechung 12 als auch insbesondere in der zurückspringenden Ausnehmung 11 eine knöcherne Brücke zwischen benachbarten Wirbelkörpern 18 aus, die mit der Außenkontur des Zwischenwirbelimplantates 1 verbunden ist und dadurch das Zwischenwirbelimplantat 1 gegen jegliche Verschiebung im Zwischenwirbelraum sichert.

Der Einführkanal 16 übernimmt somit eine Doppelfunktion einmal als Aufnahme für das Einsetzinstrument 19 und zum anderen als Zufuhr für Knochenmaterial. Dabei erstreckt sich der Einführkanal 16 aufgrund seiner schräg verlaufenden Orientierung über eine große Länge im Zwischenwirbelimplantat 1 und bietet so insbesondere dem Einsetzinstrument 19 über eine große Länge eine stabile Führung, so daß über das Einsetzinstrument 19 gegebenenfalls auch größere Kräfte auf das Zwischenwirbelimplantat 1 ausgeübt werden können, ohne daß die Gefahr einer Beschädigung des Zwischenwirbelimplantates 1 oder des Schaftes 20 des Einsetzinstrumentes 19 besteht.

Über den Einführkanal 16 kann im übrigen eine Versorgung des Knochenmaterials im Inneren der Durchbrechung 12 und auch im Inneren der zurückspringenden Ausnehmung 11 erfolgen, das Durchwachsen des Einführkanals mit Knochenmaterial führt zu einer weiteren Verbindung und Festlegung des Zwischenwirbelimplantates 1 im Zwischenwirbelraum.

## Patentansprüche

1. Zwischenwirbelimplantat (1) mit einer oberen und einer unteren ebenen Stützfläche (2, 3) zur Anlage an benachbarten Wirbelkörpern, mit einer konkaven dorsalen Längsseite (4), einer dieser gegenüberliegenden ventralen Längsseite (5), mit einer abgerundeten Kontur an den beiden die Längsseiten (4, 5) verbindenden Stirnseiten (6, 7) und mit mindestens einer das Implantat (1) von der oberen zur unteren Stützfläche (2, 3) durchsetzenden Durchbrechung (12), **dadurch gekennzeichnet, daß** mindestens ein das Implantat (1) durchsetzender Einführkanal (16) für Knochenmaterial in dem Teil einer Stirnseite (6, 7) in das Implantat (1) eintritt, der an die dorsale Längsseite (4) anschließt, und im Bereich des Mittelabschnittes (9) der ventralen Längsseite (5) wieder aus dem Implantat (1) austritt, wobei der Einführkanal (16) in einer Ebene verläuft, die zwischen der oberen Stützfläche (2) und der unteren Stützfläche (3) angeordnet ist, und gegenüber einer Längsachse des Implantates (1) in einer im wesentlichen parallel zu den Stützflächen (2, 3) verlaufenden Ebene zwischen 30° und 80° geneigt ist.

2. Implantat nach Anspruch 1, **dadurch gekennzeichnet, daß** der Einführkanal (16) eine das Implantat (1) durchsetzende Durchbrechung (12) durchquert.

3. Implantat nach einem der Ansprüche 1 oder 2, **dadurch gekennzeichnet, daß** der Einführkanal (16) an seinem ventralen Ende Befestigungselemente (17) für ein in den Einführkanal (16) eingeschobenes Instrument (19, 20) aufweist.

4. Implantat nach Anspruch 3, **dadurch gekennzeichnet, daß** das Befestigungselement (17) durch ein Innengewinde gebildet wird.

5. Implantat nach einem der voranstehenden Ansprüche, **dadurch gekennzeichnet, daß** zwei Einführkanäle (16) von verschiedenen Stirnseiten (6, 7) des Implantates (1) in eine zurückspringende Ausnehmung (11) des Mittelabschnittes (9) einmünden.

6. Implantat nach einem der voranstehenden Ansprüche, **dadurch gekennzeichnet, daß** das Implantat (1) symmetrisch zu einer senkrecht auf den Stützflächen (2, 3) und auf den Längsseiten (4, 5) stehenden Mittelebene ausgebildet ist.

7. Implantat nach einem der voranstehenden Ansprüche, **dadurch gekennzeichnet, daß** zwei Durchbrechungen (12) an den beiden Enden des Implantates (1) angrenzend an dessen Stirnseiten (6, 7) angeordnet sind.

8. Implantat nach Anspruch 7, **dadurch gekennzeichnet, daß** die Durchbrechungen (12) einen kreisförmigen Querschnitt aufweisen.

9. Implantat nach einem der voranstehenden Ansprüche, **dadurch gekennzeichnet, daß** die Stützflächen (2, 3) gegeneinander geneigt sind.

10. Implantat nach einem der voranstehenden Ansprüche, **dadurch gekennzeichnet, daß** die Stützflächen (2, 3) Vorsprünge (13, 14) tragen.

11. Implantat nach Anspruch 10, **dadurch gekennzeichnet, daß** die Vorsprünge dornförmige Spitzen (14) sind.

12. Implantat nach Anspruch 10, **dadurch gekennzeichnet, daß** die Vorsprünge Rippen (13) sind.

13. Implantat nach Anspruch 12, **dadurch gekennzeichnet, daß** die Vorsprünge (13) in Form von Rippen längs der Kanten der Durchbrechungen (12) verlaufen.

14. Implantat nach einem der voranstehenden Ansprüche, **dadurch gekennzeichnet, daß** die ventrale Längsseite (5) in ihrem mittleren Abschnitt (9) eine zurückspringende Ausnehmung (11) zur Aufnahme von Knochenmaterial aufweist.

15. Implantat nach Anspruch 14, **dadurch gekennzeichnet, daß** die Ausnehmung (11) sich über die gesamte Höhe des Implantats (1) erstreckt.

16. Implantat nach Anspruch 14 oder 15, **dadurch gekennzeichnet, daß** die Ausnehmung (11) durch einen konkaven, bogenförmig konturierten Mittelabschnitt (9) der ventralen Längsseite (5) gebildet wird.

17. Implantat nach Anspruch 16, **dadurch gekennzeichnet, daß** die an den Mittelabschnitt (9) anschließenden Seitenabschnitte (8, 10) der ventralen Längsseite (5) bogenförmig und stetig in den Mittelabschnitt (9) einmünden.

18. Implantat nach einem der voranstehenden Ansprüche, **dadurch gekennzeichnet, daß** die an den Mittelabschnitt (9) anschließenden Seitenabschnitte (8) im wesentlichen parallel zu der dorsalen Längsseite (4) verlaufen.

## Claims

1. Intervertebral implant (1) having an upper and a lower flat supporting surface (2, 3) for abutment on adjacent vertebral bodies, having a concave dorsal longitudinal side (4), a ventral longitudinal side (5) lying opposite thereto, having a rounded contour at the two end faces (6, 7) connecting the longitudinal sides (4, 5) and having at least one hole (12) penetrating the implant (1) from the upper to the lower supporting surface (2, 3), **characterized in that** at least one introduction channel (16) for bone material, which penetrates the implant (1), enters the implant (1) in the part of an end face (6, 7) adjoining the dorsal longitudinal side (4) and exits from the implant (1) in the region of the middle portion (9) of the ventral longitudinal side (5), wherein the introduction channel (16) extends in a plane disposed between the upper supporting surface (2) and the lower supporting surface (3) and is inclined between 30° and 80° relative to a longitudinal axis of the implant (1) in a plane extending substantially parallel to the supporting surfaces (2, 3).

2. Implant according to claim 1, **characterized in that** the introduction channel (16) crosses a hole (12), which penetrates the implant (1).

3. Implant according to one of claims 1 or 2, **characterized in that** the introduction channel (16) at its ventral end has fastening elements (17) for an instrument (19, 20), which is inserted into the introduction channel (16).

4. Implant according to claim 3, **characterized in that** the fastening element (17) is formed by an internal thread.

5. Implant according to one of the preceding claims, **characterized in that** two introduction channels (16) run from different end faces (6, 7) of the implant (1) into a set-back recess (11) of the middle portion (9).

6. Implant according to one of the preceding claims, **characterized in that** the implant (1) is constructed symmetrically relative to a centre plane perpendicular to the supporting surfaces (2, 3) and to the longitudinal sides (4, 5).

7. Implant according to one of the preceding claims, **characterized in that** two holes (12) are disposed at the two ends of the implant (1) adjacent to the end faces (6, 7) of the implant.

8. Implant according to claim 7, **characterized in that** the holes (12) have a circular cross section.

9. Implant according to one of the preceding claims, **characterized in that** the supporting surfaces (2, 3) are inclined relative to one another.

10. Implant according to one of the preceding claims, **characterized in that** the supporting surfaces (2, 3) carry projections (13, 14).

11. Implant according to claim 10, **characterized in that** the projections are spike-shaped points (14).

12. Implant according to claim 10, **characterized in that** the projections are ribs (13).

13. Implant according to claim 12, **characterized in that** the projections (13) in the form of ribs extend along the edges of the holes (12).

14. Implant according to one of the preceding claims, **characterized in that** the ventral longitudinal side (5) in its middle portion (9) has a set-back recess (11) for receiving bone material.

15. Implant according to claim 14, **characterized in that** the recess (11) extends over the entire height of the implant (1).

16. Implant according to claim 14 or 15, **characterized in that** the recess (11) is formed by a concave middle portion (9) of the ventral longitudinal side (5), which middle portion has an arc-shaped contour.

17. Implant according to claim 16, **characterized in that** the side portions (8, 10) of the ventral longitudinal side (5), which adjoin the middle portion (9), run in an arc-shaped manner and continuously into the middle portion (9).

18. Implant according to one of the preceding claims, **characterized in that** the side portions (8), which adjoin the middle portion (9), extend substantially parallel to the dorsal longitudinal side (4).

## Revendications

1. Implant intervertébral (1) avec des faces d'appui planes supérieure et inférieure (2, 3) destinées à venir au contact de vertèbres adjacentes, avec un côté longitudinal dorsal concave (4), un côté longitudinal ventral (5) lui faisant face, avec un contour arrondi sur les deux faces frontales (6, 7) reliant les côtés longitudinaux (4, 5) et avec au moins une cavité (12) qui passe de la face d'appui supérieure (2) à la face d'appui inférieure (3) à travers l'implant (1), **caractérisé en ce qu'**au moins un canal d'insertion (16), traversant l'implant (1) et destiné à recevoir un greffon osseux, pénètre dans l'implant (1) dans la partie d'un côté frontal (6, 7) qui est adjacente au côté longitudinal dorsal (4) et sort à nouveau de l'implant (1) dans la zone de la partie centrale (9) du côté longitudinal ventral (5), le canal d'insertion (16) étant réalisé dans un plan qui est situé entre la face d'appui supérieure (2) et la face d'appui inférieure (3) et étant incliné par rapport à un axe longitudinal de l'implant (1) entre 30° et 80° dans un plan sensiblement parallèle aux faces d'appui (2, 3).

2. Implant selon la revendication 1, **caractérisé en ce que** le canal d'insertion (16) passe à travers une cavité (12) traversant l'implant (1).

3. Implant selon la revendication 1 ou 2, **caractérisé en ce que** le canal d'insertion (16) comporte à son extrémité ventrale des éléments de fixation (17) pour un instrument (19, 20) inséré dans le canal d'insertion (16).

4. Implant selon la revendication 3, **caractérisé en ce que** l'élément de fixation (17) est formé par un taraudage.

5. Implant selon l'une quelconque des revendications précédentes, **caractérisé en ce que** deux canaux d'insertion (16) partant de différents côtés frontaux (6, 7) de l'implant (1) débouchent dans un évidement (11) en retrait dans la partie centrale (9).

6. Implant selon l'une quelconque des revendications précédentes, **caractérisé en ce que** l'implant (1) est réalisé symétriquement à un plan médian situé perpendiculairement sur les faces d'appui (2, 3) et sur les côtés longitudinaux (4, 5) .

7. Implant selon l'une quelconque des revendications précédentes, **caractérisé en ce que** deux cavités (12) sont réalisées sur les deux extrémités de l'implant (1) de manière adjacente aux côtés frontaux (6, 7) de celui-ci.

8. Implant selon la revendication 7, **caractérisé en ce que** les cavités (12) ont une section ronde.

9. Implant selon l'une quelconque des revendications précédentes, **caractérisé en ce que** les faces d'appui (2, 3) sont inclinées l'une par rapport à l'autre.

10. Implant selon l'une quelconque des revendications précédentes, **caractérisé en ce que** les faces d'appui (2, 3) portent des protubérances (13, 14).

11. Implant selon la revendication 10, **caractérisé en ce que** les protubérances sont des pointes (14) en forme d'épines.

12. Implant selon la revendication 10, **caractérisé en ce que** les protubérances sont des nervures (13).

13. Implant selon la revendication 12, **caractérisé en ce que** les protubérances (13) en forme de nervures s'étendent le long des bords des cavités (12).

14. Implant selon l'une quelconque des revendications précédentes, **caractérisé en ce que** le côté longitudinal ventral (5) comporte dans sa partie centrale (9) un évidement (11) en retrait destiné à recevoir du tissu osseux.

15. Implant selon la revendication 14, **caractérisé en ce que** l'évidement (11) s'étend sur toute la hauteur de l'implant (1).

16. Implant selon la revendication 14 ou 15, **caractérisé en ce que** l'évidement (11) est formé par une partie centrale (9) concave à contour curviligne dans le côté longitudinal ventral (5).

17. Implant selon la revendication 16, **caractérisé en ce que** les parties latérales (8, 10) du côté longitudinal ventral (5), adjacentes à la partie centrale (9), débouchent avec une forme curviligne et régulière dans la partie centrale (9).

18. Implant selon l'une quelconque des revendications précédentes, **caractérisé en ce que** les parties latérales (8), adjacentes à la partie centrale (9), sont sensiblement parallèles au côté longitudinal dorsal (4).
